# EUROPEAN PATENT APPLICATION

(11) **EP 1 964 518 A1**
(43) Date of publication of application: **03.09.2008**
(21) Application number: 06834986.9
(22) Date of filing: 13.12.2006
(51) Int. Cl.: A61B 8/08

(54) **MEDICAL ULTRASONIC APPARATUS HAVING IRRADIATION POSITION-CONFIRMING FUNCTION**

(30) Priority: 14.12.2005 JP 2005360501
(71) Applicant: Teijin Pharma Limited, Chiyoda-ku, Tokyo 100-0013 (JP)
(72) Inventor: TAKABAYASHI, Junichi, Hino-shi, Tokyo 191-0065 (JP); DEGUCHI, Tsuneo, Tokyo 100-0011 (JP)
(74) Representative: HOFFMANN EITLE
(86) International application number: PCT/JP2006/325316
(87) International publication number: WO 2007/069775

(57) **Abstract**

The present invention is a medical ultrasound device in which an ultrasound pulse is irradiated to a treatment or examination region, provided with ultrasound-wave irradiating means, signal receiving means for receiving a reflected wave of the ultrasound irradiated from the ultrasound irradiating means, signal recording means (memory) for recording a received signal, and comparing means (comparison operation element) for comparing a recorded signal and the received signal. By the device of the present invention, an appropriate treatment or examination position can be checked with high reproducibility.

## Description

### Technical Field

The present invention relates to a medical ultrasound device. The present invention also relates to a device having a function to check if an ultrasound is appropriately irradiated to an intended position using an ultrasound and particularly to an ultrasound fracture treatment device.

### Background Art

In case of applying medical devices to treatment or examination, it is sometimes important to accurately grasp a position of an affected area. As a method of obtaining in-vivo information noninvasively, visualization techniques such as conventional X-ray, MRI, CT scan or diagnostic ultrasound and the like are often used. There are numerous devices for grasping the position of an affected area in a body as above. However, huge costs or specialized techniques are required to use these devices, and it is difficult for an individual patient to use such devices. In addition, specialized knowledge is indispensable to determine an appropriate treatment position from the visualized information.

Particularly with ultrasound fracture treatment devices used for accelerating bone union, treatment is conducted by irradiating a uniform ultrasound to an affected area from an ultrasound transducer. The ultrasound is used for diagnosis and treatment in general as a safe and simple physical therapy. On the other hand, there is also a problem that an irradiation direction of the ultrasound is easily changed by attaching position or angle of the transducer, and in this case, the ultrasound cannot be irradiated to an affected area appropriately. Particularly at ultrasound irradiation to a fracture site without plaster bandage, it is extremely difficult to fix the transducer, and it is hard for a patient to attach the ultrasound transducer at a position and an angle prescribed by a medical institution, and the irradiation position of the transducer might be displaced.

In case of position or angle displacement of the transducer, optimal treatment cannot be conducted and treatment efficiency is remarkably lowered. Therefore, development of a method for checking a correct irradiation position is an important object for detecting irradiation position displacement of a transducer and to check an accurate irradiation position.

Japanese Patent No. 2790777, for example, discloses an art relating to a bone-position detection device for detecting a bone position by irradiating an ultrasound to a living body and by receiving its reflected wave. This art describes a method of detecting a bone position in a bone evaluating device but if position displacement or the like of the transducer occurs, for example, it is possible to detect a bone surface but it is impossible to detect ultrasound irradiation displacement from an affected area for treatment determined in the medical institution.

Japanese Translation Patent Publication No. 10-509605 discloses an ultrasound treatment device in which an ultrasound for examination is irradiated to a bone before treatment and intensity of the ultrasound for treatment is optimized by receiving its reflected wave. Japanese Patent Laid-Open No. 2000-325383 is also a patent relating to optimization of the intensity of ultrasound for treatment. These patents are ultrasound treatment devices not using an ultrasound for examination but irradiating an ultrasound used for treatment and receiving its reflected wave. By these arts, the intensity of the ultrasound for treatment can be changed according to a treatment location, but ultrasound irradiation displacement from the affected area for treatment caused by positional displacement or the like of the transducer cannot be detected, either.

In order to prevent deterioration in treatment or examination efficiency, realization of checking a correct ultrasound irradiation position is an indispensable art in medical ultrasound irradiation devices.

### Disclosure of the invention

The present invention solves a problem that attachment of a medical device at a position and an angle as prescribed by a medical institution is difficult for a patient when an examination or treatment is to be given using the medical device.

As a result of intensive consideration of the problem, the inventors have found a method of checking if a position is in exact agreement with prescribed position or not by specifying an irradiation position by receiving a reflected wave when an ultrasound is irradiated to an affected area, by recording a received signal at that time and by comparing the signal with a received signal received similarly at a subsequent examination or treatment.

More specifically, when prescription is set in advance at a medical institution or the like, a reflected wave from each tissue such as skin, muscle, bone or the like when an ultrasound is irradiated from an ultrasound transducer to an affected area is received from the transducer and recorded in advance as a prescribed irradiation position (reference signal), and at subsequent treatment, a received wave obtained at irradiation of the ultrasound to the affected area is compared / analyzed with the reference signal so as to check if the ultrasound can be irradiated at a prescript position appropriately.

That is, the present invention is:
(1) a medical ultrasound device in which an ultrasound pulse is irradiated to a treatment or examination region, the device comprising a first transducer having an ultrasound irradiation function, a second transducer having a function for receiving a reflected wave of the ultrasound irradiated from the first transducer, a memory for recording a received signal, and a comparison operation element for comparing a recorded signal and the received signal;
(2) a medical ultrasound device described in (1), in which the treatment or examination region is a fracture site;
(3) a medical ultrasound device described in (1) or (2), in which the second transducer receives a plurality of reflected waves reflected from the periphery of the treatment or examination region;
(4) a medical ultrasound device described in any of (1) to (3), in which the memory records at least one of signals of intensity, receiving time and pulse width of the received signal;
(5) a medical ultrasound device described in (4), in which the comparison operation element is provided with a display portion that calculates at least one of parameters of intensity attenuation ratio, delay rate of receiving time, and fluctuation rate of pulse width of the received signal based on at least one of signals of intensity, receiving time and pulse width of the received signal, compares the newly calculated parameters with parameters recorded in advance and displays a parameter comparison result;
(6) a medical ultrasound device described in any of (1) to (5), in which the ultrasound pulse is a low-intensity ultrasound pulse of 100 mW/cm² or less;
(7) a medical ultrasound device described in any of (1) to (6), in which if a comparison result of the comparison operation element is out of a preset threshold value, the display portion displays an alarm signal;
(8) a medical ultrasound device described in any of (1) to (7), in which a position adjustment mechanism is provided for adjusting an irradiation position of the first transducer if a comparison result of the comparison operation element is out of a preset threshold value;
(9) a medical ultrasound device described in any of (1) to (8), in which the first transducer and the second transducer are constituted by the same ultrasound transducer;
(10) a medical ultrasound device for irradiating an ultrasound pulse to a treatment or examination region, comprising ultrasound irradiating means, signal receiving means for receiving a reflected wave of the ultrasound irradiated from the ultrasound irradiating means, signal recording means for recording a received signal, and comparing means for comparing the recorded signal and the received signal;
(11) a medical ultrasound device described in (10), in which the treatment or examination region is a fracture site;
(12) a medical ultrasound device described in (10) or (11), in which the signal receiving means receives a plurality of reflected waves reflected from the periphery of the treatment or examination region;
(13) a medical ultrasound device described in any of (10) to (12), in which the signal recording means records at least one of signals of intensity, receiving time and pulse width of the received signal;
(14) a medical ultrasound device described in (13), in which the comparing means is provided with display means that calculates at least one of parameters of intensity attenuation ratio, delay rate of receiving time, and fluctuation rate of pulse width of the received signal based on at least one of signals of intensity, receiving time and pulse width of the received signal, compares the newly calculated parameters with parameters recorded in advance and displays a parameter comparison result;
(15) a medical ultrasound device described in any of (10) to (14), in which the ultrasound pulse is a low-intensity ultrasound pulse of 100 mW/cm² or less;
(16) a medical ultrasound device described in any of (10) to (15), in which if a comparison result of the comparing means is out of a preset threshold value, the display means displays an alarm signal;
(17) a medical ultrasound device described in any of (10) to (16), in which position adjusting means is provided for adjusting an irradiation position of the ultrasound irradiating means if a comparison result of the comparing means is out of a preset threshold value;
(18) a medical ultrasound device described in any of (10) to (17), in which the ultrasound irradiating means and the signal receiving means are constituted by the same ultrasound transducer;
(19) a control method for a medical ultrasound device, in which an ultrasound pulse is irradiated to a treatment or examination region from a first transducer, a reflected wave reflected from the periphery of the treatment or examination region is received by a second transducer, and at least one of signals of intensity, receiving time and pulse width of the received signal is recorded by a memory;
(20) a control method for a medical ultrasound device described in (19), in which the treatment or examination region is a fracture site;
(21) a control method for a medical ultrasound device described in (19) or (20), in which the second transducer receives a plurality of reflected waves reflected from the periphery of the treatment or examination region;
(22) a control method for a medical ultrasound device described in any of (19) to (21), in which at least one of parameters of intensity attenuation ratio, delay rate of receiving time, and fluctuation rate of pulse width of the received signal is calculated based on at least one of signals of intensity, receiving time and pulse width of the received signal, the newly calculated parameters are compared with parameters recorded in a memory in advance and a parameter comparison result is displayed on a display portion;
(23) a control method for a medical ultrasound device described in any of (19) to (22), in which the ultrasound pulse is a low-intensity ultrasound pulse of 100 mW/cm² or less;
(24) a control method for a medical ultrasound device described in any of (19) to (23), in which if a comparison result of the comparison operation element is out of a preset threshold value, an alarm signal is displayed on the display portion;
(25) a control method for a medical ultrasound device described in any of (19) to (24), in which if a comparison result of the comparison operation element is out of a preset threshold value, a position adjustment mechanism adjusts an irradiation position of the first transducer;
(26) a control method for a medical ultrasound device described in any of (19) to (25), in which the first transducer and the second transducer are constituted by the same ultrasound transducer;
(27) a control method for a medical ultrasound device characterized in that an ultrasound pulse is irradiated from ultrasound irradiating means to a treatment or examination region , a reflected wave reflected from the periphery of the treatment or examination region is received by signal receiving means, and at least one of signals of intensity of the received signal, receiving time, and pulse width is recorded by recording means.
(28) a control method for a medical ultrasound device described in (27), wherein the treatment or examination region is a fracture site;
(29) a control method for a medical ultrasound device described in (27) or (28), in which the signal receiving means receives a plurality of reflected waves reflected from the periphery of the treatment or examination region;
(30) a control method for a medical ultrasound device described in any of (27) to (29), in which at least one of parameters of intensity attenuation ratio, delay rate of receiving time, and fluctuation rate of pulse width of the received signal is calculated based on at least one of signals of intensity, receiving time and pulse width of the received signal, the newly calculated parameters are compared with parameters recorded in advance and a parameter comparison result is displayed on display means;
(31) a control method for a medical ultrasound device described in any of (27) to (30), in which the ultrasound pulse is a low-intensity ultrasound pulse of 100 mW/cm² or less;
(32) a control method for a medical ultrasound device described in any of (27) to (31), in which if a comparison result of the comparing means is out of a preset threshold value, an alarm signal is displayed on the display means;
(33) a control method for a medical ultrasound device described in any of (27) to (32), in which if a comparison result of the comparing means is out of a preset threshold value, an irradiation position of the ultrasound irradiating means is adjusted by position adjusting means; and
(34) a control method for a medical ultrasound device described in any of (27) to (33), in which the ultrasound irradiation means and the signal receiving means are constituted by the same ultrasound transducer.

### Brief Description of the Drawings

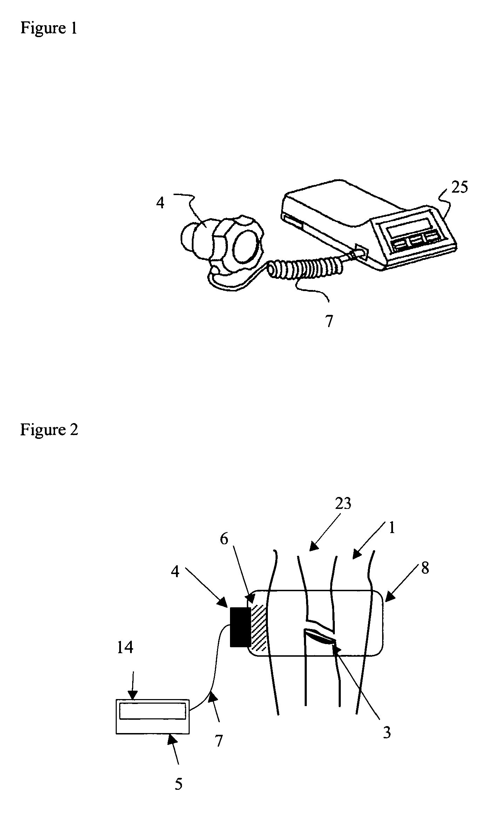

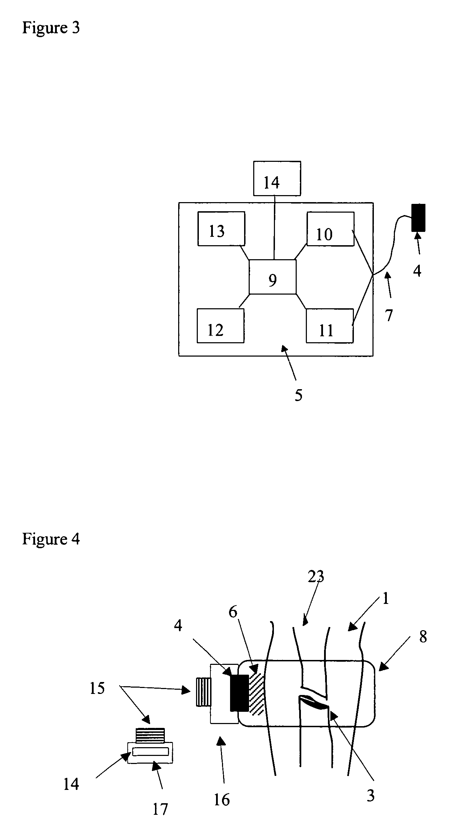

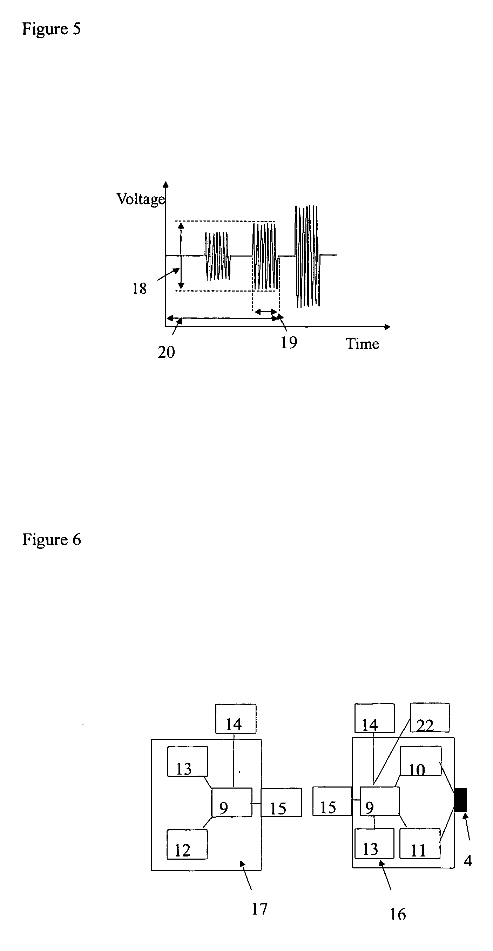

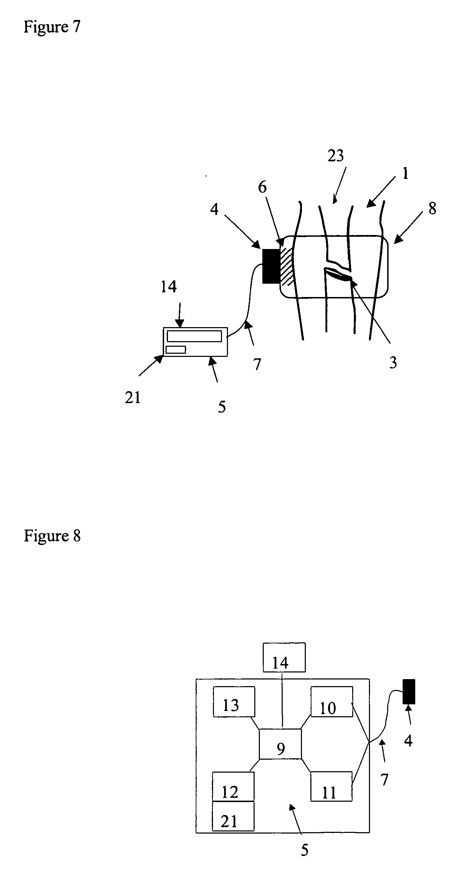

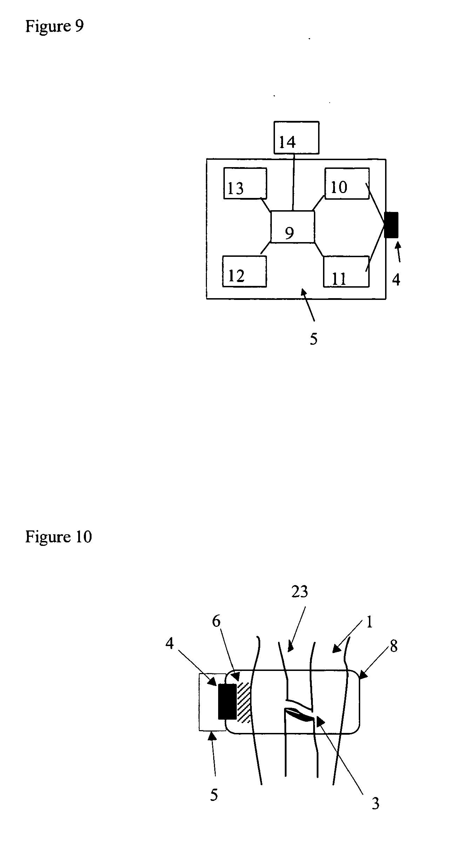

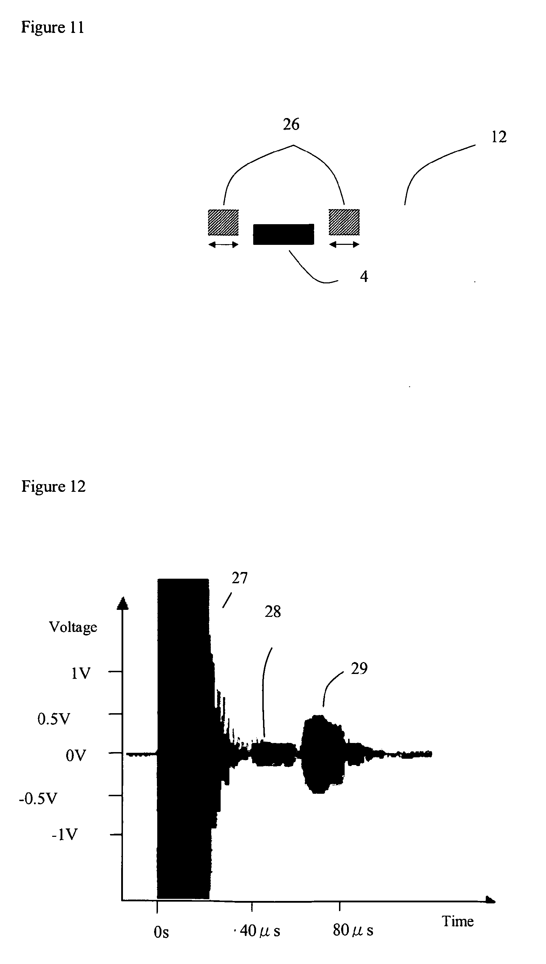

Fig. 1 shows the embodiment of the medical ultrasound device. Fig. 2 shows the state that the medical ultrasound device is attached to the treated area. Fig. 3 is the block diagram of the fracture treatment device. Fig. 4 shows the state that the medical ultrasound device is attached to the treated area. Fig. 5 is an analysis example of a received signal of the present invention. Fig. 6 is the block diagram of the wireless fracture treatment device. Fig. 7 is an example 3 of the fracture treatment device of the present invention. Fig. 8 is the block diagram of the fracture treatment device. Fig. 9 is the block diagram of the medical ultrasound device in which the fracture treatment device and the transducer are integrated. Fig. 10. shows the state that the integrated medical ultrasound device is attached to the treated area. Fig. 11 shows an embodiment of fixing means having a position adjustment function. Fig. 12 is a signal example obtained when the medical ultrasound device of the present invention is applied to a femur.

### Best Mode for Carrying Out the Invention

The medical ultrasound device of the present invention sends an ultrasound for examination from an ultrasound transducer to an affected area in the course of prescribing treatment by medical equipment. The transducer used for sending is also used as a receiving terminal so that only a signal included in a specific range of signal arrival time is selectively detected among ultrasound signals reflected in a body and recorded in the treatment device.

When conducting treatment, an ultrasound for examination is sent out before treatment and a reflected wave is detected. Intensity, arrival time, and duration of the signal are compared with the recorded signal and analyzed.

If a detection signal equivalent to the signal recorded at prescription is obtained, the medical device is installed at an applicable location, and the ultrasound for examination is switched to an ultrasound for treatment so as to give treatment. If a detection signal different from the signal recorded at the prescription is obtained or if a detection signal is not obtained, the ultrasound for examination is sent out again and the detection signal is analyzed. This is repeated till an appropriate signal is obtained.

By the above method, the present invention can confirm that the ultrasound transducer is attached at a prescribed position. In addition, the present invention can confirm that the ultrasound is irradiated to a prescribed position.

Fig. 1 shows an example of the medical ultrasound device for embodying the present invention. The present invention is not limited to this application example or illustrated examples. The medical ultrasound device shown in Fig. 1 comprises a controller 25 for carrying out sending control of the ultrasound and analysis of a received signal and a transducer 4 connected through a cable 7. The transducer 4 is used for sending and receiving of an ultrasound. The transducer 4 may be constituted by a plurality of transducers conducting sending and receiving separately. If constituted by a plurality of transducers, the receiving side may be a sensor that can receive an ultrasound such as a microphone (hydrophone), pressure sensor or the like with high sensitivity instead of transducer. All the examples below describe a device constituted by a single transducer, but the present invention is not limited by those examples.

More specifically, an example of an ultrasound fracture treatment device of the present invention is shown in Fig. 2.

When setting prescription in a medical institution such as a hospital at the beginning of treatment, an installation position of a transducer is determined by X-ray diagnostic imaging and the like so that an ultrasound for treatment hits a fracture site 3 of a femur 23. The transducer 4 is attached by transducer fixing means 8 at a prescribed position. At this time, an ultrasound gel 6 is applied as an ultrasound propagation substance between the transducer 4 and a soft tissue 1. An ultrasound signal for examination from the fracture treatment device 5 is transmitted to the transducer 4 through a cable 7 so as to irradiate ultrasound which frequency can be set at 1 to 5 MHz or preferably 1.5 MHz; burst width can be set at 5 to 200 µs or preferably 5 to 70 µs, or more preferably 20 µs; repetition cycle can be set at 10 Hz to 10 kHz or preferably 1 kHz; time average and spatial average of ultrasound output can be set at 0.75 to 30 mW/cm² or preferably 3 mW/cm². An irradiation time can be easily adjusted by control means and the irradiation time may be changed according to a purpose, but irradiation continues for 1 to 10 seconds, for example. The longer the irradiation time, the better the accuracy becomes, therefore the irradiation time is set at approximately 10 seconds, for example at setting of prescription, or 1 to 5 seconds, for example, or preferably approximately 2 seconds at home, considering convenience.

Since acoustic impedance is different among a soft tissue 1 (fat, muscle and the like) and bones in a body respectively, the ultrasound wave is reflected at a boundary where the acoustic impedance is different. The transducer 4 receives the ultrasound reflected at each boundary, and at this time, the transducer generates electric signal. The generated signal is sent to the fracture treatment device 5 through the cable 7, the signal is analyzed, and its result is displayed on display means (display portion) 14. If a medical staff determines that it is an appropriate result, this is recorded in recording means (memory) 12 and handled as a reference signal. A patient conducts treatment using the device in which the signal is recorded. Upon reception of the signal, there is a possibility that a noise signal might be received due to external factors such as body movement, electric field, magnetic field and the like.

For example, in the diagnostic ultrasound device used widely in public, duration of ultrasound with 1 µs or less is used in order to improve image resolution, but in this case, the device is susceptible to noise and it is difficult to grasp accurately an irradiation position of the transducer, which is the object to be solved by the present invention. That is, in case of examination for diagnosis, a signal can be received on the real time basis all the time to be visualized as an image, and even mixture of a noise in the received signal would not make a big problem. Also, as in Japanese Patent Laid-Open No. 2005-318921 as an example of a related art, technologies to reduce noise have been actively studied by applying filtering operation or the like to the received signal. However, the purpose of filtering operation is to construct an image that professionals can interpret easily. On the other hand, for the purpose of the present invention that an irradiation position of the transducer in the ultrasound irradiation device for treatment is to be accurately grasped, comparison of the received signals at the irradiation position is important, and improvement of signal accuracy is more important. Thus, in the present invention, the accuracy of the reflected signal from the target can be improved by sending a signal having a constant pulse width or duration, a pulse width of 20 µs, for example, and removing influence of noise.

Non-destructive inspection used in the industrial field is distinguished from the present invention as the ultrasound image diagnosis. In the non-destructive inspection, similarly to the ultrasound image diagnosis, real-time imaging is carried out by a reflected signal obtained by the transducer. Based on the image, diagnosis of disease or flaws is made by trained professionals. On the other hand, in the present invention, by making comparison with a recorded signal, an irradiated portion is determined. By this arrangement, reproducible positioning can be realized regardless of expertise of a user. Here, the ultrasound fracture treatment device is used as an example, but the present invention is an art that can be applied to medical equipment requiring prescription of an appropriate position among medical equipment used for examination or treatment and can be realized noninvasively, that is, safely using a low-intensity ultrasound pulse.

For example, when a patient conducts treatment at home, the transducer 4 is mounted at an attachment position prescribed by a medical institution, an ultrasound for examination is irradiated, and a detected signal can be compared with a reference signal determined by the medical institution. As shown in Fig. 5, the signal comparison is made automatically by control means 9 provided with comparing means (comparison operation element) using intensity of received signal 18, time width of received signal 19, and delay time of received signal 20. More specifically, by calculating a signal attenuation rate, transmission speed, and signal duration from the intensity of received signal 18 and the time width of received signal 19 of the reflected signal at the irradiation region, thickness of tissue such as fat, fascia, muscle, bone and the like is determined, respectively, and moreover by acquiring the signal attenuation rate and time to detection from the intensity of received signal 18 and the delay time of received signal 20, the distances between each tissue and the irradiation position and relative positions are determined and recorded.

A detection method according to the present invention will be described using Fig. 5. Fig. 5 shows a schematic waveform of a reflected signal when an ultrasound wave for examination with 1.5 MHz, burst width of 20 µs, repetition cycle of 1 kHz, and spatial average and time average of an ultrasound output of 3 mW/cm² is irradiated to a femur. In Fig. 5, the first signal with the shortest interval between ultrasound irradiation and signal receiving is expected to be reflected at the boundary between fat and muscle, the second signal is expected to be reflected at the boundary between two types of muscle (fascia), and a third signal with the longest delay time to receiving is reflected at the boundary between muscle and bone. The signal intensity is determined by a difference in acoustic impedance at the reflection boundary and attenuation relating to a distance over which the ultrasound wave is propagated. A signal reflected from the boundary between fat and muscle with a small difference in the acoustic impedance is smaller and the signal reflected from the boundary between muscle and bone with the acoustic impedance different by twice or more is larger. A reflected signal from a shallow boundary face has small influence of attenuation, while the influence of attenuation is larger on the reflected signal from a deep boundary face in a body. By the above signal processing, the thickness of the respective tissues and / or relative positional relations can be obtained. In the example in Fig. 5, a region irradiating the ultrasound wave is known to be a layer of fat, muscle, another muscle, and bone. Moreover, using the time width of received signal for carrying out identification of continuity of signals, integration of signals exceeding a threshold value or analysis of an average value of signal intensity, for example, an accidental noise can be removed. Also, if material with different acoustic impedance is present nearby, reflected signals from a plurality of substances are synthesized and received, but it is possible to dissolve the synthesized signal using the time width of received signal and the acoustic impedance characteristics at the respective tissues and to make detection as the respective reflected signals.

Fig. 12 shows an example of the waveform of transmitted received signal. Fig. 12 shows the transmitted signal 27 and the received signal 28 when the ultrasound for examination with 1.5 MHz, burst width of 20 µs, repetition cycle of 1 kHz, and spatial average and time average of the ultrasound output of 3 mW/cm² is irradiated to a femur.. In this example, the received signal 28 is determined as reflection by fascia and another received signal 29 as reflection by bone.

By calculating thickness, relative position and distance from the skin of each tissue the specific irradiation position and comparing them with the values stored in the memory, the irradiation position is judged to be a right position prescribed by the medical institution. If a position or angle of the transducer is different from pre-determined ones, there is a possibility of increase, decrease or loss of signal intensity, delay in a detection time or the like in Fig. 5, for example. At this time, a degree of differences between calculated values and stored values, which determines if displacement occurs, can be preset for each patient appropriately, but ±10% for signal intensity and ±5% for arrival time can be specified as an error range, for example. If the reflected wave signal has displacement from the reference signal apart from the preset error range, the fact that the treatment position is different from a position of initial setting is notified to the patient by the display means (display portion) 14. If it is within the error range of the reference signal, the ultrasound for examination is changed to an ultrasound for treatment, and the ultrasound with a frequency of 1.5 MHz, burst width of 200 µs, repetition cycle of 1 kHz, and spatial average and time average of the ultrasound output of 30 mW/cm² is irradiated from the transducer so as to start treatment. An optimal ultrasound wave irradiation can be continued by regularly checking and re-setting by the patient of the irradiation position at the medical institution.

Figure 3 shows the block diagram of the fracture treatment device.

In the fracture treatment device 5, nature of the ultrasound irradiated by the transducer 4 is determined by a signal transmitted from the oscillating circuit 10 through the cable 7. An electric signal generated when the transducer 4 receives ultrasound is detected by the receiving circuit 11 through the cable 7 and stored in the recording means (memory) 12. Power supply means 13 is built-in power supply or means that can receive external power supply and is a driving source of the ultrasound fracture treatment device. The display means (display portion) 14 is means for providing information such as state of the fracture treatment device 5, irradiation situation of the ultrasound and the like. The above components are adjusted by the control means 9, and the control means 9 is also provided with a function as comparing means (comparison operation element) of the signal.

Fig. 4 shows an application example of a wireless-type ultrasound fracture treatment device using femur fracture treatment as an example. By making the cable 7 connecting the fracture treatment device 5 and the transducer 4 wireless, it becomes possible to freely arrange the transducer 4. Moreover, though clothes had to be taken off during treatment depending on a treatment portion, it becomes easy to treat having the clothes on by a wireless ultrasound-wave irradiation device 16.

The ultrasound signal at the treatment is transmitted to the transducer 4 through wireless communicating means 15. The signal received by the transducer 4 is sent to the wireless ultrasound irradiation device 16, the signal is sent to a wireless fracture treatment device 17 using the wireless communicating means 15, and the result can be displayed by the display means (display portion) 14.

Moreover, at this time, if displacement of an irradiation position is detected, the irradiation position can be adjusted by position adjusting means (position adjustment mechanism). The irradiation position adjustment can be carried out manually or automatically. The automatic adjustment is conducted by irradiation-position automatic adjusting means 22, and the irradiation-position automatic adjusting means 22 is controlled by the control means 9. The irradiation-position automatic adjusting means 22, for example can be realized by using a conductive polymer material 26 as fixing means 8, which expands or contracts by applying voltage. A specific example is shown in Fig. 11. This is an example in which a belt is used as the fixing means 8, and an ultrasound can be irradiated to a lower direction in Fig. 11. By constructing one or more portion of the fixing means 8 by the conductive polymer material 26 capable of expansion / contraction and by making adjustment by the irradiation-position automatic adjusting means 22, an installation position of the transducer 4 on a body surface can be adjusted by expansion / contraction of the conductive polymer material 26. Specifically, expansion / contraction in an arrow direction in Fig. 11 is possible. A distance that can be adjusted by this expansion / contraction depends on the type or size of the conductive polymer material, but expansion / contraction by approximately 1 mm to 5 cm is sufficient. By using the conductive polymer material 26 at a position on the back face (direction into which the ultrasound wave is not irradiated) of the transducer 4, a force to press the transducer 4 onto the body surface can be also adjusted. Moreover, by increasing expansion / contraction spots by this expanding / contracting conductive polymer material 26, finer adjustment and optimization of the ultrasound irradiation position can be realized. The plurality of expansion / contraction spots can be expanded / contracted at the same time or independently. An example of expanding / contracting conductive polymer material 26 may include polypyrrole.

Fig. 6 shows the configuration of the wireless fracture treatment device.

The nature of the ultrasound irradiated by the transducer 4 is determined by a sending circuit in the wireless ultrasound irradiation device 16. An electric signal generated when the transducer 4 senses the ultrasound is detected by the receiving circuit 11. The wireless communicating means 15 is placed in the wireless ultrasound irradiation device 16 and the wireless fracture treatment device 17 and enables to transmit and receive the signal. The signal detected by the receiving circuit is sent to the recording means (memory) 12 in the wireless fracture treatment device 17 through the wireless communicating means 15 and stored therein. The power supply means 13 is built-in power supply or means that can be provided from external power supply and is a driving source of the wireless ultrasound irradiation device and the wireless fracture treatment device, respectively. The display means (display portion) 14 provides information such as states of the wireless ultrasound irradiation device 16 and the wireless fracture treatment device 17, communication state, irradiation state of the ultrasound wave and the like. When displacement in the irradiation position is detected, the position of the transducer 4 can be automatically adjusted by the irradiation-position automatic adjusting means 22. The above elements are controlled by the control means 9.

Fig. 7 shows an example of an ultrasound fracture treatment device with recording media applied to femur fracture treatment as an example.

The signal received when prescription is set at start of treatment can be stored in a recording media 21. For example, a patient can bring back only the recording media 21 for treatment at home and set it at the fracture treatment device owned by the patient at treatment.

At the start of the treatment, the recording media 21 in which a reference signal is stored is set at the fracture treatment device, an ultrasound wave for examination is irradiated at an attachment position prescribed by the medical institution, and comparison is made between a signal detected similarly as shown in the example in Fig. 2 and the reference signal determined by the medical institution. By regularly checking and re-setting the irradiation position at the medical institution, the patient can continue optimal ultrasound-wave irradiation. At this time, by bringing only the recording media 21 to the medical institution, simpler treatment control is enabled.

Fig. 8 shows an example of device configuration.

In the fracture treatment device 5, the nature of the ultrasound irradiated by the transducer 4 is determined by a signal transmitted from the oscillating circuit 10 through the cable 7. The electric signal generated when the transducer 4 senses the ultrasound is detected by the receiving circuit 11 through the cable 7 and stored in the recording media 21 at the recording means (memory) 12. The power supply means 13 is built-in power supply or means that can be provided from external power supply and is a driving source for the ultrasound fracture treatment device. The display means (display portion) 14 is means for providing information such as a state of the fracture treatment device 5, irradiation state of the ultrasound and the like. The above elements are adjusted by the control means 9.

Fig. 9 shows an example of device components.

The nature of the irradiated ultrasound wave is determined by a signal from the oscillating circuit 10 to the transducer 4 integrated with the fracture treatment device 5. The electric signal generated when the transducer 4 senses the ultrasound is detected by the receiving circuit 11 and stored in the recording means (memory) 12. The power supply means 13 is built-in power supply or means that can be provided by from external power supply and is a driving source for the ultrasound fracture treatment device. The display means (display portion) 14 is means for providing information such as a state of the fracture treatment device 5, irradiation state of the ultrasound and the like. The above elements are adjusted by the control means 9, and the control means 9 is also provided with a function as the comparing means (comparison operation element) of the signal.

Fig. 10 shows an example of an integral ultrasound fracture treatment device applied to femur fracture treatment.

In Fig. 10 cable 7 is eliminated and the ultrasound fracture treatment device 5 and the transducer 4 are integrated. Since the ultrasound fracture treatment device 5 itself can be used by fixing it on a body, the treatment device can be used regardless of the installation location.

The medical ultrasound device of the present invention can be used for medical applications not limited to fracture treatment. For example, it can be used for examination or treatment of muscle, fat, organ and the like at a specific position, not limited to the bone. Moreover, it can be used in combination with other medical examination equipment or treatment equipment, and examination or treatment with high reproducibility for an intended position can be provided. For example, by using a laser treatment device after checking a position by the medical ultrasound device of the present invention, a laser beam with property of traveling in straight lines can be irradiated to an intended portion efficiently. Other than that, in the case of puncture, an intended portion can also be positioned with high reproducibility by the medical ultrasound device of the present invention. The medical ultrasound device of the present invention enables position adjustment with high reproducibility when medical equipment requiring positioning is to be used.

### Industrial Applicability

By using the medical ultrasound device of the present invention, a position such as irradiation to the target position and the like can be confirmed with high reproducibility. As a result, examination or treatment is expected to be carried out accurately and efficiently. Further, by notifying a patient that examination or treatment is being carried out at an appropriate position, improvement effect of treatment compliance can be expected.

## Claims

1. A medical ultrasound device in which an ultrasound pulse is irradiated to a treatment or examination region, the device comprising a first transducer having an ultrasound irradiation function, a second transducer having a function for receiving a reflected wave of the ultrasound irradiated from the first transducer, a memory for recording a received signal, and a comparison operation element for comparing a recorded signal and the received signal.

2. The medical ultrasound device according to claim 1, wherein the treatment or examination region is a fracture site.

3. The medical ultrasound device according to claim 1 or 2, wherein the second transducer receives a plurality of reflected waves reflected from the periphery of the treatment or examination region.

4. The medical ultrasound device according to any of claims 1 to 3, wherein the memory records at least one of signals of intensity, receiving time and pulse width of the received signal.

5. The medical ultrasound device according to claim 4, wherein the comparison operation element is provided with a display portion that calculates at least one of parameters of intensity attenuation ratio, delay rate of receiving time, and fluctuation rate of pulse width of the received signal based on at least one of signals of intensity, receiving time and pulse width of the received signal, compares the newly calculated parameters with parameters recorded in advance and displays a parameter comparison result.

6. The medical ultrasound device according to any of claims 1 to 5, wherein the ultrasound pulse is a low-intensity ultrasound pulse of 100 mW/cm² or less.

7. The medical ultrasound device according to any of claims 1 to 6, wherein if a comparison result of the comparison operation element is out of a preset threshold value, the display portion displays an alarm signal.

8. The medical ultrasound device according to any of claims 1 to 7, wherein a position adjustment mechanism is provided for adjusting an irradiation position of the first transducer if a comparison result of the comparison operation element is out of a preset threshold value.

9. The medical ultrasound device according to any of claims 1 to 8, wherein the first transducer and the second transducer are constituted by the same ultrasound transducer.

10. A medical ultrasound device for irradiating an ultrasound pulse to a treatment or examination region, comprising ultrasound irradiating means, signal receiving means for receiving a reflected wave of the ultrasound irradiated from the ultrasound irradiating means, signal recording means for recording a received signal, and comparing means for comparing the recorded signal and the received signal.

11. The medical ultrasound device according to claim 10, wherein the treatment or examination region is a fracture site.

12. The medical ultrasound device according to claim 10 or 11, wherein the signal receiving means receives a plurality of reflected waves reflected from the periphery of the treatment or examination region.

13. The medical ultrasound device according to any of claims 10 to 12, wherein the signal recording means records at least one of signals of intensity, receiving time and pulse width of the received signal.

14. The medical ultrasound device according to claim 13, wherein the comparing means is provided with display means that calculates at least one of parameters of intensity attenuation ratio, delay rate of receiving time, and fluctuation rate of pulse width of the received signal based on at least one of signals of intensity, receiving time and pulse width of the received signal, compares the newly calculated parameters with parameters recorded in advance and displays a parameter comparison result.

15. The medical ultrasound device according to any of claims 10 to 14, wherein the ultrasound pulse is a low-intensity ultrasound pulse of 100 mW/cm² or less.

16. The medical ultrasound device according to any of claims 10 to 15, wherein if a comparison result of the comparing means is out of a preset threshold value, the display means displays an alarm signal.

17. The medical ultrasound device according to any of claims 10 to 16, wherein position adjusting means is provided for adjusting an irradiation position of the ultrasound irradiating means if a comparison result of the comparing means is out of a preset threshold value.

18. The medical ultrasound device according to any of claims 10 to 17, wherein the ultrasound irradiating means and the signal receiving means are constituted by the same ultrasound transducer.

19. A control method for a medical ultrasound device, **characterized in that** an ultrasound pulse is irradiated to a treatment or examination region from a first transducer, a reflected wave reflected from the periphery of the treatment or examination region is received by a second transducer, and at least one of signals of intensity, receiving time and pulse width of the received signal is recorded by a memory.

20. The control method for the medical ultrasound device according to claim 19, wherein the treatment or examination region is a fracture site.

21. The control method for the medical ultrasound device according to claim 19 or 20, wherein the second transducer receives a plurality of reflected waves reflected from the periphery of the treatment or examination region.

22. The control method for the medical ultrasound device according to any of claims 19 to 21, wherein at least one of parameters of intensity attenuation ratio, delay rate of receiving time, and fluctuation rate of pulse width of the received signal is calculated based on at least one of signals of intensity, receiving time and pulse width of the received signal, the newly calculated parameters are compared with parameters recorded in a memory in advance and a parameter comparison result is displayed on a display portion.

23. The control method for the medical ultrasound device according to any of claims 19 to 22, wherein the ultrasound pulse is a low-intensity ultrasound pulse of 100 mW/cm² or less.

24. The control method for the medical ultrasound device according to any of claims 19 to 23, wherein if a comparison result of the comparison operation element is out of a preset threshold value, an alarm signal is displayed on the display portion.

25. The control method for the medical ultrasound device according to any of claims 19 to 24, wherein if a comparison result of the comparison operation element is out of a preset threshold value, a position adjustment mechanism adjusts an irradiation position of the first transducer.

26. The control method for the medical ultrasound device according to any of claims 19 to 25, wherein the first transducer and the second transducer are constituted by the same ultrasound transducer.

27. The control method for the medical ultrasound device **characterized in that** an ultrasound pulse is irradiated from ultrasound irradiating means to a treatment or examination region, a reflected wave reflected from the periphery of the treatment or examination region is received by signal receiving means, and at least one of signals of intensity of the received signal, receiving time, and pulse width is recorded by recording means.

28. The control method for the medical ultrasound device according to claim 27, wherein the treatment or examination region is a fracture site.

29. The control method for the medical ultrasound device according to claim 27 or 28, wherein the signal receiving means receives a plurality of reflected waves reflected from the periphery of the treatment or examination region.

30. The control method for the medical ultrasound device according to any of claims 27 to 29, wherein at least one of parameters of intensity attenuation ratio, delay rate of receiving time, and fluctuation rate of pulse width of the received signal is calculated based on at least one of signals of intensity, receiving time and pulse width of the received signal, the newly calculated parameters are compared with parameters recorded in advance and a parameter comparison result is displayed on display means.

31. The control method for the medical ultrasound device according to any of claims 27 to 30, wherein the ultrasound pulse is a low-intensity ultrasound pulse of 100 mW/cm² or less.

32. The control method for the medical ultrasound device according to any of claims 27 to 31, wherein if a comparison result of the comparing means is out of a preset threshold value, an alarm signal is displayed on the display means.

33. The control method for a medical ultrasound device according to any of claims 27 to 32, wherein if a comparison result of the comparing means is out of a preset threshold value, the position adjusting means adjusts an irradiation position of the ultrasound irradiating position.

34. The control method for the medical ultrasound device according to any of claims 27 to 33, wherein the ultrasound irradiation means and the signal receiving means are constituted by the same ultrasound transducer.
